# EUROPEAN PATENT SPECIFICATION

(11) **EP 2 055 333 B1**
(45) Date of publication and mention of the grant of the patent: **18.08.2010**
(21) Application number: 08253500.6
(22) Date of filing: 28.10.2008
(51) Int. Cl.: A61M 5/158, A61M 5/32, A61M 25/00

(54) **Flexible cannula comprising a nitinol strip jacketed by a flexible tube for medical applications**
Flexible Kanüle aus einem Nitinolstreifen überzogen mit einem flexiblen Schlauch für medizinische Anwendungen
Canule flexible comprenant une lame de nitinol couverte d'un tuyau flexible pour des applications médicales

(30) Priority: 29.10.2007 US 983530 P; 30.10.2007 US 983651 P; 31.10.2007 US 984066 P
(43) Date of publication of application: 06.05.2009
(73) Proprietor: Lifescan, Inc., Milpitas, CA 95035 (US)
(72) Inventor: Krulevitch, Peter, Pleasanton, CA 94566 (US); Cichocki, Frank, Easton, PA 18045 (US); Olson, Lorin P., Scotts Valley, CA 95066 (US)
(74) Representative: Tunstall, Christopher Stephen

(56) References cited:
- EP-A- 0 615 768
- WO-A-02/05866
- WO-A-98/17337
- WO-A-02/081013
- WO-A-2005/068000
- US-A1- 2002 072 720

## Description

### BACKGROUND OF THE INVENTION

Field of the Invention

The present invention relates, in general, to medical devices and, in particular, to flexible conduits and associated medical devices and methods.

Description of Related Art

A variety of medical devices employ conduits for navigating through the body and accessing specific target sites in order to perform diagnostic, therapeutic, and surgical procedures. For example, flexible cannulas inserted by rigid needles are conventionally employed for the infusion of therapeutic agents (e.g., insulin).

WO02/05866 A2 describes a catheter-based deployment system for deploying cellular material into the heart muscle. The deployment system includes a guiding catheter, a needle assembly, with a needle tip, capable of sliding within the guiding catheter. The needle tip has an opening in communication with a lumen within the needle assembly. In operation the needle tip penetrates the muscle wall. Cellular material may be deployed into the muscle wall via the operation of a push rod, through the lumen and out of the opening within the needle tip.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings, in which like numerals indicate like elements, of which:
FIG. 1A is a simplified cross-sectional depiction of a flexible cannula according to an exemplary embodiment of the present invention;
FIG. 1B is a simplified cross-sectional depiction of the flexible cannula of FIG. 1A taken along line B-B of FIG. 1A;
FIG. 1C is a simplified cross-sectional depiction of the flexible cannula of FIG. 1A taken along line C-C of FIG. 1A;
FIGs 2A and 2B are simplified depictions, side and end views respectively, of an elongated Nitinol strip with a channel therein as can be employed in embodiments of present invention;
FIG. 3 is a simplified cross-sectional depiction of another elongated Nitinol strip as can be employed in embodiments of the present invention with exemplary dimensions in inches indicated thereon;
FIG. 4 is a simplified cross-sectional depiction of another elongated Nitinol strip as can be employed in embodiments of the present invention with exemplary dimensions in inches indicated thereon;
FIG. 5 is a simplified depiction of another elongated Nitinol strip as can be employed in embodiments of the present invention with exemplary dimensions in inches indicated thereon;
FIG. 6 is a simplified depiction of yet another elongated Nitinol strip as can be employed in embodiments of the present invention;
FIGs. 7A-7D are simplified depictions of other elongated Nitinol strips as can be employed in embodiments of the present invention with exemplary dimensions in inches indicated thereon;
FIGs. 8A and 8B are simplified depictions of an isotropic etching process as can be employed to manufacture flexible cannula according to the present invention;
FIGs. 9A and 9B are simplified depictions of a medical device according to embodiments of the present invention before deployment of an integrated flexible cannula and after deployment, respectively; and
FIGs. 10, 11, 12 and 13 are various simplified views of another medical device according to an embodiment of the present invention that includes a flexible cannula guide.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict selected exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. In addition, as used herein, the terms "patient", "host" and "subject" refer to any human or animal subject and are not intended to limit the systems or methods to human use, although use of the subject invention in a human patient represents a preferred embodiment.

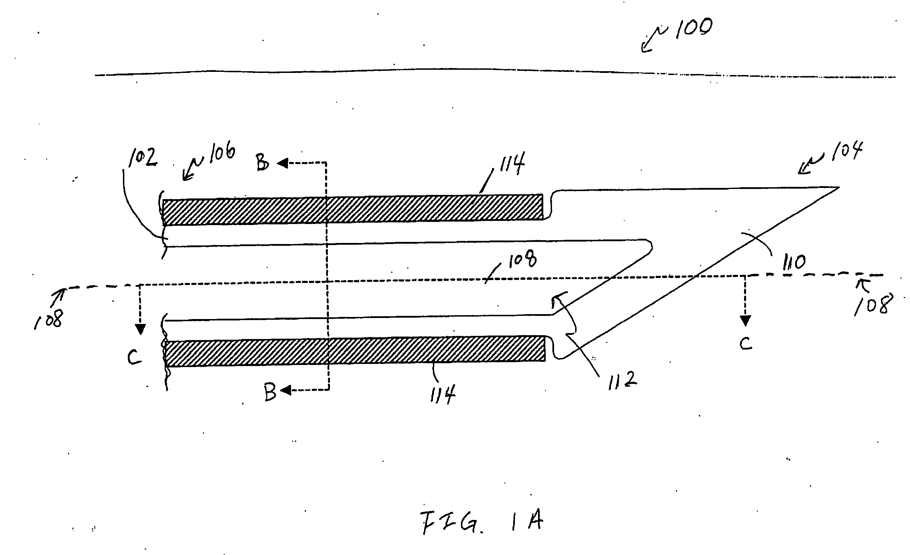

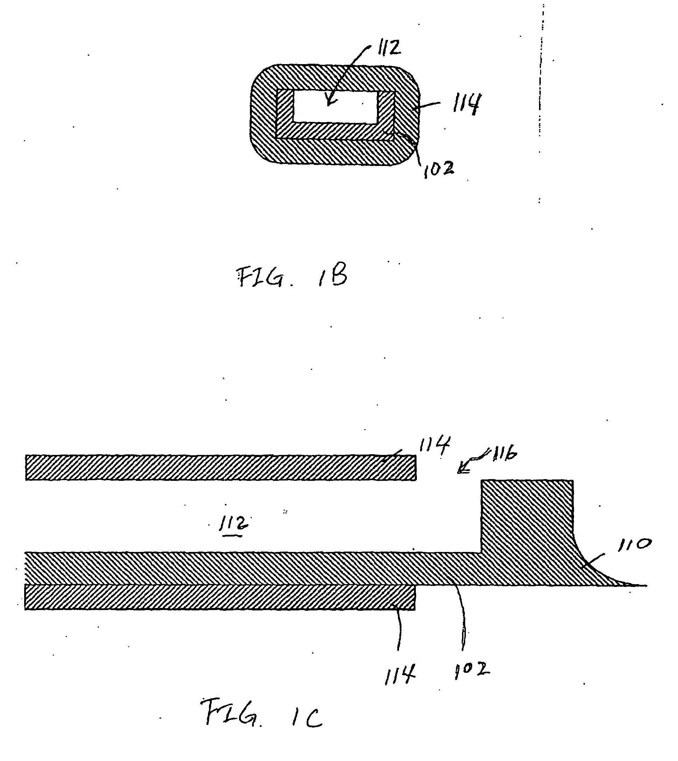

FIGs. 1A, 1B and 1C depict a flexible cannula 100 according to an embodiment of the present invention. Flexible cannula 100 includes an elongated strip 102 (such as a flexible Nitinol strip or other suitable highly flexible strip) with a distal end 104, a proximal end 106 (shown only the direction of where the proximal end will be) a longitudinal axis 108 (depicted by a dashed line) running from distal end 104 to proximal end 106, a sharp head 110 extending from distal end 104 and a channel 112 formed (for example, etched) therein. Channel 112 is dispositioned at least partially along, or parallel to, the longitudinal axis 108.

Flexible medical device 100 also includes a flexible tube 114 at least partially fixedly jacketing the elongated strip between distal end and the proximal end, the channel and flexible tube defining a conduit. Channel 112 extends partially into sharp head 110 such that a conduit opening 116 on the side of the flexible cannula 100 is defined.

If desired, flexible tube 114 can extend past proximal end 106 and be configured to provide a fluid-tight connection to associated medical device components (such as infusion components). Moreover, if desired, the flexible cannula can be coated with a lubricious material to facilitate insertion into a user's target site.

Various other aspects of flexible cannula according to the present invention including methods for their use, manufacture and employment in integrated medical devices are described below.

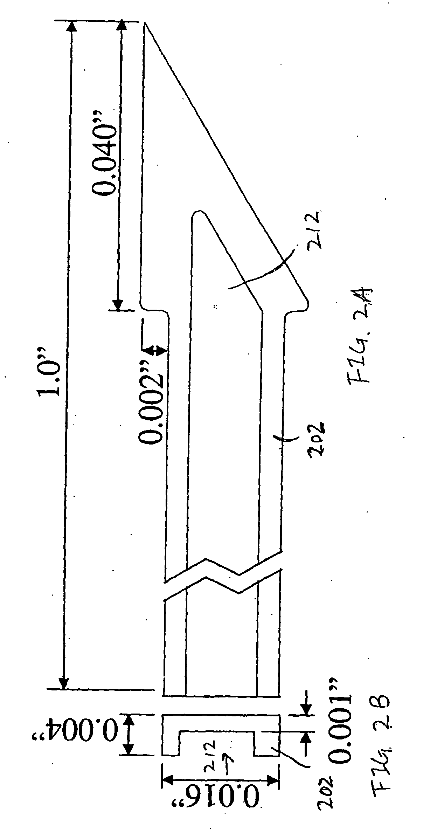

FIGs 2A and 2B are simplified depictions, side and end views respectively, of an elongated Nitinol strip 202 with a channel 212 therein as can be employed in embodiments of present invention. FIGs, 2A and 2B include exemplary, non-limiting, dimensions in inches.

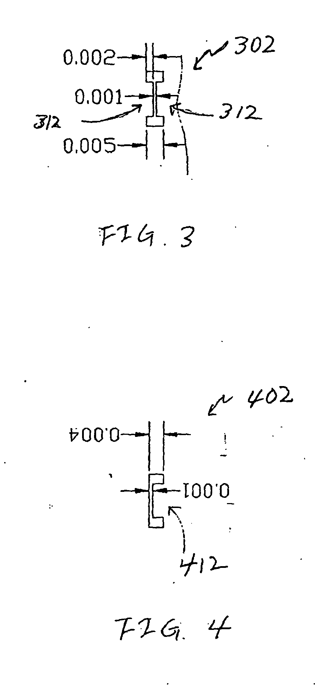

FIG. 3 is a simplified cross-sectional depiction of another elongated Nitinol strip 302 with two channels 312 as can be employed in embodiments of the present invention with exemplary dimensions in inches indicated thereon. The two channels form an elongated Nitinol strip with an "H-shaped" cross-section. The presence of two channels provides conduit redundancy or for the provision of different fluids in each conduit (for example, insulin and Smylin). Moreover, the H-shaped cross-section provides additional flexibility in comparison to a C-shaped cross-section as depicted in FIG. 4 and described below).

FIG. 4 is a simplified cross-sectional depiction of another elongated Nitinol strip 402 with channel 412 therein as can be employed in embodiments of the present invention with exemplary dimensions in inches indicated thereon.

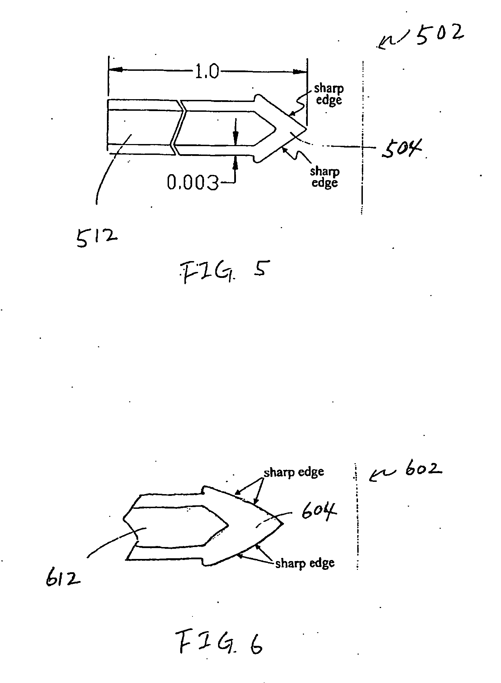

FIG. 5 is a simplified depiction of another elongated Nitinol strip 502 with channel 512 and sharp head 504 as can be employed in embodiments of the present invention with exemplary dimensions in inches indicated thereon. FIG. 6 is a simplified depiction of yet another elongated Nitinol strip 602 with sharp head 604 and channel 612 as can be employed in embodiments of the present invention.

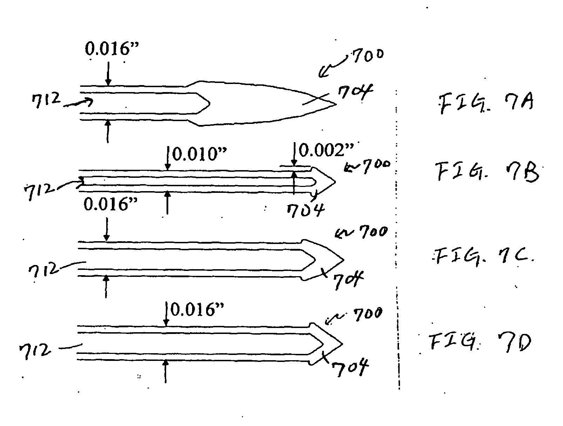

FIGs. 7A-7D are simplified depictions of other elongated Nitinol strips 700 with sharp heads 704 and channels 712 as can be employed in embodiments of the present invention with exemplary dimensions in inches indicated thereon.

Methods for manufacturing a flexible cannula according to embodiments of the present invention include etching a channel into an elongated Nitinol strip and forming a sharp head on a distal end of the elongated Nitinol strip. The methods also include subsequently jacketing the flat elongated Nitinol strip with a flexible tube such that the flexible tube and channel define a conduit.

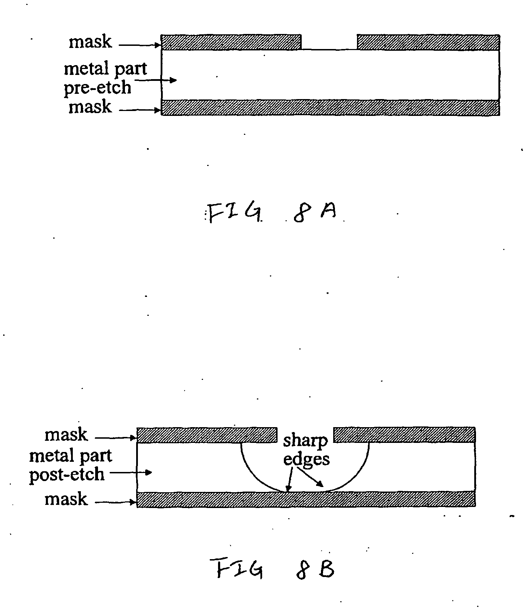

FIGs. 8A and 8B are simplified depictions of an isotropic etching process as can be employed to manufacture flexible cannulae according to the present invention. Channels employed in embodiments of the present invention can be formed using, for example, any suitable etching technique known to those skilled in the art including isotropic chemical etching techniques. Isotropic etching employs a masking layer and results in undercutting of the masking layer, producing sidewalls with a semi-circular cross-section having sharp edges at the bottom of the etched surface after removing the masking layer, as shown in FIGs. 8A and 8B.

If it is desired to have a curved elongated strip (such as a curved elongated Nitinol strip), curled sheet material can be used instead of flat sheet. In this case, the etch mask must be properly aligned with the curvature of the sheet. Alternatively, the strips can be curled in a secondary manufacturing operation.

A channel can be etched on one side of an elongated strip (referred to as a "C" shaped cross section, see FIG. 2B for example) or on both sides (referred to as an "H" shaped cross section, see FIG. 3) of the strip. It is also possible to etch more than one channel on one or both sides of an elongated strip. Etching more than one channel provides some redundancy, in case one of the channels becomes blocked, or the additional channels could be used to deliver different drugs, such as insulin and Symlin.

The sharp head of the strip with the penetrating sharp edges is made wider than the remainder of the elongated strip such that when a flexible tube (for example, a polymer jacket) is placed around the strip to form the conduit, the leading edge of the flexible tube fits the shoulders on the head. This decreases the frontal profile of the flexible cannula, reducing insertion force and preventing the flexible tube from catching on the incised insertion point in a user's target site, which can lead to "accordioning" of the polymer. The etched channel extends into the sharp head to provide an opening beyond the flexible tube for the fluid to flow into the user's target site. Positioning the opening on the side of the sharp head beneficially reduces the chance of blocking the channel from coring of tissue during insertion. Commonly used masking techniques such as corner compensation may be used where the head of the strip meets the body in order to obtain the proper shape in the etched part.

Many sharp head configurations (see FIGs. 7A through 7D) are possible since etching allows for the shape of the sharp head to be designed independently of the body.

A flexible cannula according to an exemplary embodiment of the present invention can be formed, for example, using a flexible conduit comprising an etched elongated Nitinol strip (with a sharp head) surrounded by a heat shrunk PTFE polymer jacket (with a recovered ID .012" max, recovered wall 0.002", expanded ID 0.048" min from Zeus). In this embodiment, the etched channel in the elongated Nitinol strip extends beyond the PTFE jack to allow the fluid to exit. In addition, the heat shrink PTFE tubing tapers down at the juncture of the sharp head, which will facilitate insertion into a user's target site.

Medical devices according to embodiments of the present invention include a flexible cannula that has an elongated Nitinol strip with a distal end, a proximal end, a longitudinal axis running from the distal end to the proximal end, a sharp head extending from the distal end, and a channel etched therein. Alternatively, the channel can be formed by using other suitable methods, such as stamping. Moreover, the channel is dispositioned along, or parallel to, the longitudinal axis. The flexible cannula also has a flexible tube at least partially jacketing the elongated Nitinol strip between the distal end and the proximal end, with the channel and the flexible tube defining a conduit. The medical device also includes an insertion mechanism configured to insert a portion of the flexible cannula, including the sharp head, into a user's target site such that the conduit provides fluid communication to the target site.

The flexible cannula employed in such medical devices has been described above (for example, with respect to FIGs. 1A through 8B). Exemplary embodiments of the integrated insertion mechanism are described below. In this respect it should be noted that the flexible cannula is integrated with the insertion mechanism in that the flexible cannula is not removed, separated or discarded during use.

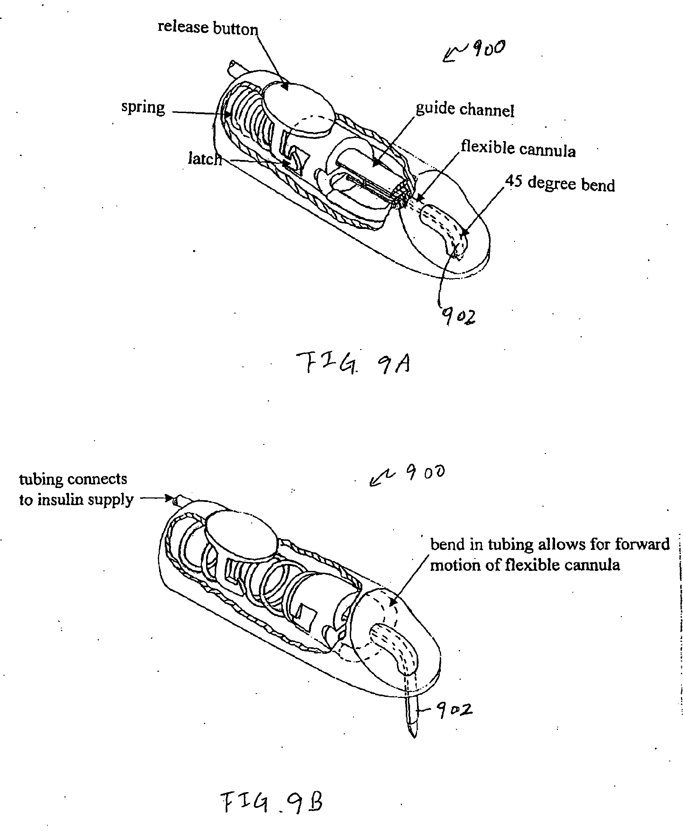

FIGs. 9A and 9B show simplified depictions of a medical device 900 according to embodiments of the present invention before deployment of an integrated flexible cannula and after deployment, respectively. Medical device 900 includes a flexible cannula 902 and an insertion mechanism 904. Various components of the insertion mechanism (i.e., a firing release button, firing spring, latch, and guide channel are illustrated in FIGs. 9A and 9B). As noted in these FIGs., the flexible cannula flexible tubing portion can be connected to, for example, an insulin supply source.

Medical device 900 can be fired by pressing the release button to release the latch, or, alternatively, it can be automatically fired by an electromechanical switch (not shown). Medical device 900 can be provided to a user spring-loaded as shown in FIG. 9A. The device is held in the loaded position by the latch, which can be moved out or the way by pressing the release button. The flexible cannula resides inside a channel that is approximately parallel to the surface of the user's skin, and bends at a 45 degree angle towards the user's body at the tip. The flexible cannula is normally straight, but follows the 45 degree bend because of the superelastic properties of Nitinol. Other angles than 45 degrees can be used for the deployment angle.

To use the medical device, the adhesive backing (not shown) is removed from the bottom of the medical device, and the medical device is applied (adhered) to the user's skin. Because the medical device requires minimal dexterity to handle and is small compared to other infusion sets with auto inserters, it is easily applied to any location on the body that can be touched by the user, for example the top of the buttocks, back of the arm, side, abdomen, and thigh (back, front, or side).

To deploy (insert) the medical device flexible conduit, the user presses on the release button, which releases the latch and allows the spring to fire the medical device. The flexible cannula follows the channel guide, travels through the 45 degree bend, and inserts across the user's skin. The user can press the device with one or more fingers, the thumb, the palm, or any part of the hand or arm that is convenient. Very limited dexterity or force is required to activate the insertion mechanism. Alternatively, an electromechanical mechanism can be used to automatically fire the device, eliminating the requirement for the user to press a release button.

The medical device as shown in FIGs. 9A and 9B includes a coil of tubing which allows the flexible cannula to move forward while connected to the insulin supply. Alternatively, a connector can be located at the back of the medical device that slides forward with the flexible cannula. After deployment, tubing can be attached to the back of the device via a suitable connector in order to connect to the insulin supply.

The horizontal configuration of medical device 900 disclosed has numerous advantages including:
- The low profile design is well-suited for integration into a patch pump
- The Nitinol strip can be manufactured flat, which reduces manufacturing steps
- The device has a simple design with few components
- The spring design is very straight-forward and the spring force is easy to adjust
- The flexible Nitinol/polymer cannula (i.e., flexible cannula) will not buckle when penetrating the skin because it is supported along its entire length
- The device comes with the spring pre-loaded so the user is not psychologically intimidated by the force required to load the spring
- The device makes minimal noise during deployment due to the small mass of the moving part. Damping materials can be incorporated into the device to further reduce noise.
- The user does not see a needle before, during, or after insertion, making the device psychologically easy to insert

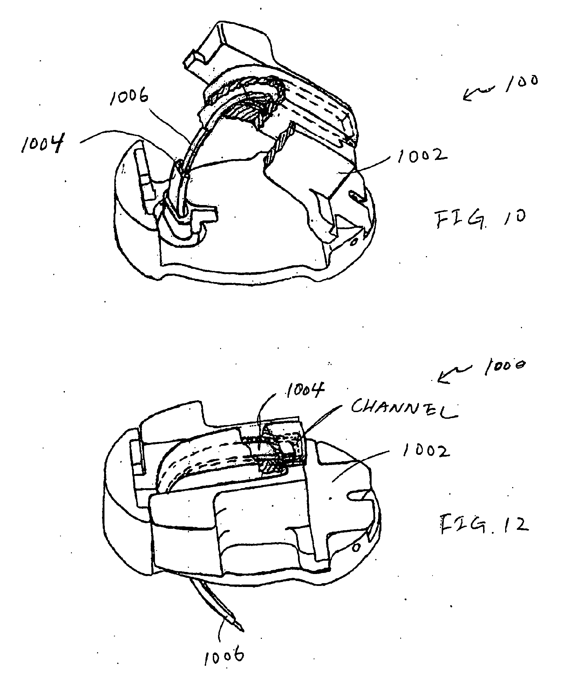

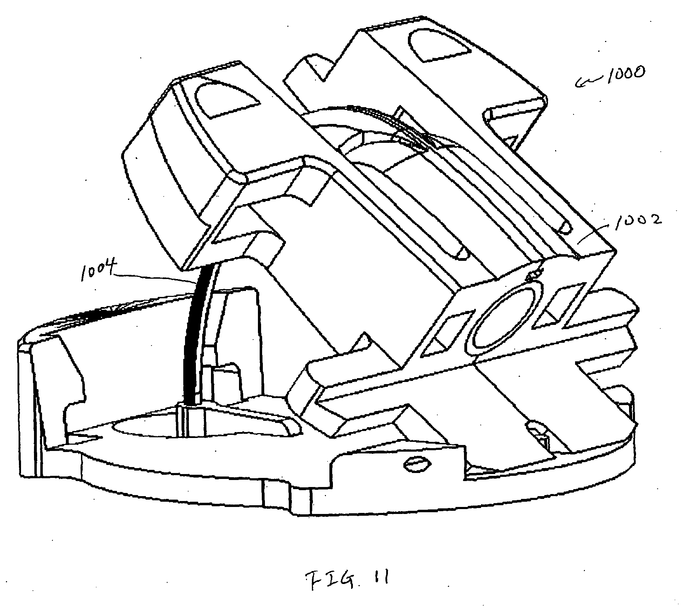

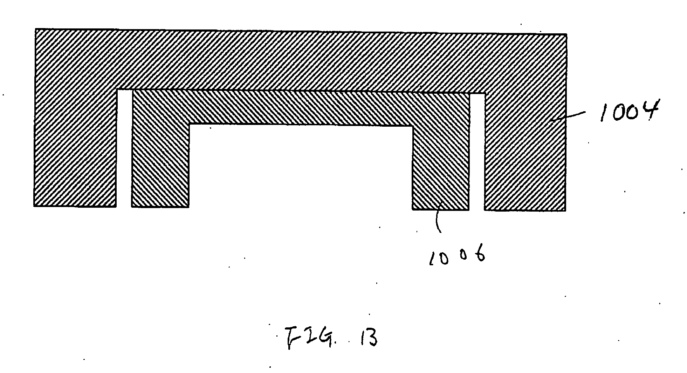

FIGs. 10, 11, 12 and 13 are various simplified views of medical device 1000 according to an embodiment of the present invention. Medical device 1000 includes an insertion mechanism 1002 (with a flexible anti-buckling cannula guide 1004) and an integral flexible cannula 1006. FIGs. 10 and 11 depict the medical device prior to deployment (insertion) of the flexible cannula into a user's target site. FIG. 12 depicts the medical device after deployment. FIG. 13 is a simplified cross-sectional depiction of the flexible anti-buckling cannula cooperating with the flexible cannula guide).

Referring to FIGs, 10, 11, 12 and 13, medical device 1000 includes a flexible anti-buckling cannula guide 1004 to prevent the integral flexible cannula 1006 from buckling during insertion into a user's target site. The configuration of medical device 1000 provides anti-buckling support to the flexible cannula along its entire length.

Flexible anti-buckling cannula guide 1004 is formed, for example, of Nitinol and has a channel (or alternatively a groove) configured to operatively cooperate with the flexible cannula (see, for example, FIG. 13). Prior to deployment, flexible cannula is positioned inside the channel of the anti-buckling cannula guide (see, for example, FIG. 10).

When the insertion force is applied at the end of the flexible cannula during use (and after the medical device has been adhered to a user by, for example, the use of an adhesive layer on the bottom of the medical device) the flexible cannula bows toward the anti-buckling guide, pressing against it. The Nitinol anti-buckling cannula guide limits the extent to which the flexible cannula bends, thus preventing the flexible cannula from buckling. As the insertion mechanism closes (i.e., transitions from the position of FIG. 10 to the position of FIG. 11 via manual user force), the flexible cannula pierces user's the skin and enters the subcutaneous tissue (not shown in the FIGs.). At the same time, the Nitinol anti-buckling cannula guide travels upwards into a channel located in the insertion mechanism (labeled as such in FIG. 12) and bends (see FIG. 12). Because Nitinol is superelastic, it bends easily without kinking.

While the focus of this disclosure has been medical devices and methods related to insulin delivery, embodiments of the present invention are also useful for delivery of other drugs or biological agents such as DNA or cells, insertion of sensors, or extraction of samples such as blood, interstitial fluid, or tissue.

Methods are also disclosed for inserting a flexible cannula into a user's target site that includes adhering a medical device to a user with the medical device having a flexible cannula and an integrated insertion mechanism.

Moreover, the flexible cannula has an elongated Nitinol strip with a distal end, a proximal end, a longitudinal axis running from the distal end to the proximal end, a sharp head extending from the distal end, and a channel etched therein In addition, the channel is dispositioned along, or parallel to, the longitudinal axis. The medical device flexible conduit also includes a flexible tube at least partially fixedly jacketing the elongated Nitinol strip between the distal end and the proximal end, the channel and flexible tube defining a conduit. The insertion mechanism is configured to insert a portion of the flexible cannula including the sharp head into a user's target site such that the conduit provides fluid communication to the target site. The method also includes inserting the flexible cannula into the user's target site.

The sharp head of the flexible cannula remains in the target site during use of the medical device (for example during the administration of insulin) and is only removed when the entire flexible cannula is removed from the target site. Since the flexible cannula is highly flexible (for example, being formed of Nitinol and a flexible polymer tube), it can remain inserted without undue pain or discomfort during use.

## Claims

1. A flexible cannula (100) comprising:
an elongated Nitinol strip (102) with:
a distal end (104);
a proximal end (106);
a longitudinal axis (108) running from the distal end to the proximal end;
a sharp head (110) extending from the distal end; and
a channel (112) formed therein, the channel dispositioned at least partially parallel to the longitudinal axis and
a flexible tube (114) at least partially fixedly jacketing the elongated Nitinol strip between the distal end and the proximal end, the channel and flexible tube defining a conduit, wherein the channel extends partially into the sharp head such that a conduit opening (116) on the side of the flexible cannula is defined.

2. The flexible cannula of claim 1, wherein the channel is dispositioned at least partially along the longitudinal axis.

3. A medical device comprising:
the flexible cannula of claim 1, and
an insertion mechanism configured to insert a portion of the flexible cannula, including the sharp head, into a user's target site such that the conduit provides fluid communication to the target site.

4. The medical device of claim 3, wherein the channel is dispositioned along the longitudinal axis.

5. A method for manufacturing a flexible cannula (100) as defined in claim 1 comprising:
forming a channel (112) into an elongated Nitinol strip (102);
forming a sharp head (110) on a distal end (104) of the elongated Nitinol strip; and fixedly jacketing the flat elongated Nitinol strip with a flexible tube (114) such that the flexible tube and channel define a conduit, and wherein the channel extends partially into the sharp head such that a conduit opening (116) on the side of the flexible cannula is defined.

6. The method for manufacturing a flexible cannula of claim 5, wherein the channel is formed by etching.

7. The method for manufacturing a flexible cannula of claim 5, wherein the channel is formed by stamping.

## Patentansprüche

1. Flexible Kanüle (100), die aufweist:
einen länglichen Nitinol-Streifen 102) mit:
einem distalen Ende (104),
einem proximalen Ende (106;
einer Längsachse (108), die von dem distalen Ende zu dem proximalen Ende verläuft;
einen scharfen Kopf (110), der sich von dem distalen Ende her erstreckt; und
einen darin gebildeten Kanal (112), wobei der Kanal wenigstens teilweise parallel zu der Längsachse angeordnet ist, und
einen flexiblen Schlauch (114), der wenigstens teilweise fest den länglichen Nitinol-Streifen zwischen dem distalen Ende und dem proximalen Ende ummantelt, wobei der Kanal und der flexible Schlauch eine Leitung definieren, wobei sich der Kanal teilweise in den scharfen Kopf derart erstreckt, dass eine Öffnung (116) der Leitung auf der Seite der flexiblen Kanüle definiert wird.

2. Flexible Kanüle nach Anspruch 1, bei der der Kanal wenigstens teilweise entlang der Längsachse angeordnet ist.

3. Medizinische Vorrichtung, die aufweist:
die flexible Kanüle nach Anspruch 1 und
einen Einführmechanismus, der so gestaltet ist, dass er einen Teil der flexiblen Kanüle, einschließlich des scharfen Kopfes, in einen Zielort des Benutzers einführt, so dass die Leitung für eine fluidische Verbindung zu dem Zielort sorgt.

4. Medizinische Vorrichtung nach Anspruch 3, bei der der Kanal entlang der Längsachse angeordnet ist.

5. Verfahren zum Herstellen einer flexiblen Kanüle (100) nach Anspruch 1, das aufweist:
Formen eines Kanals (112) in einen länglichen Nitinol-Streifen (102);
Bilden eines scharfen Kopfes (110) auf einem distalen Ende (104) des länglichen Nitinol-Streifens; und
festes Ummanteln des flachen länglichen Nitinol-Streifens mit einem flexiblen Schlauch (114), derart, dass der flexible Schlauch und der Kanal eine Leitung definieren, und wobei sich der Kanal teilweise in den scharfen Kopf erstreckt, so dass eine Öffnung (116) der Leitung auf der Seite der flexiblen Kanüle definiert wird.

6. Verfahren zum Herstellen einer flexiblen Kanüle nach Anspruch 5, bei der der Kanal durch Ätzen geformt wird.

7. Verfahren zum Herstellen einer flexiblen Kanüle nach Anspruch 5, bei der der Kanal durch Prägen geformt wird.

## Revendications

1. Canule flexible (100) comprenant :
■ une bande de Nitinol allongée (102) avec :
■ une extrémité distale (104) ;
■ une extrémité proximale (106) ;
■ un axe longitudinal (108) s'étendant de l'extrémité distale à l'extrémité proximale ;
■ une tête pointue (110) s'étendant à partir de l'extrémité distale ; et
■ un canal (112) formé à l'intérieur de celle-ci, le canal étant positionné au moins partiellement parallèle à l'axe longitudinal, et
■ un tube flexible (114) recouvrant au moins partiellement de manière fixe la bande de Nitinol allongée entre l'extrémité distale et l'extrémité proximale, le canal et le tube flexible définissant un conduit, dans laquelle le canal s'étend partiellement dans la tête pointue de sorte que l'on définit une ouverture de conduit (116) sur le côté de la canule flexible.

2. Canule flexible selon la revendication 1, dans laquelle le canal est disposé au moins partiellement le long de l'axe longitudinal.

3. Dispositif médical comprenant :
■ une canule flexible selon la revendication 1, et
■ un mécanisme d'insertion configuré pour insérer une partie de la canule flexible, comprenant la tête pointue, dans un site cible d'un utilisateur de sorte que le conduit fournit la communication de fluide jusqu'au site cible.

4. Dispositif médical selon la revendication 3, dans lequel le canal est disposé le long de l'axe longitudinal.

5. Procédé pour fabriquer une canule flexible (100) selon la revendication 1, comprenant les étapes consistant à :
■ former un canal (112) dans une bande de Nitinol allongée (102) ;
■ former une tête pointue (110) sur une extrémité distale (104) de la bande de Nitinol allongée ; et
■ recouvrir de manière fixe la bande de Nitinol allongée plate avec un tube flexible (114) de sorte que le tube flexible et le canal définissent un conduit, et dans lequel le canal s'étend partiellement dans la tête pointue de sorte que l'on définit une ouverture de conduit (116) sur le côté de la canule flexible.

6. Procédé pour fabriquer une canule flexible selon la revendication 5, dans lequel le canal est formé par gravure.

7. Procédé pour fabriquer une canule flexible selon la revendication 5, dans lequel le canal est formé par estampage.
